Europäisches Patentamt

European Patent Office

Office européen des brevets

(19)

(11) Numéro de publication: **0 293 535 B1**

(12) **FASCICULE DE BREVET EUROPEEN**

(45) Date de publication de fascicule du brevet:
**10.04.91**

(51) Int. Cl.⁵: **A61K 7/48**, A61K 31/23

(21) Numéro de dépôt: **87401274.3**

(22) Date de dépôt: **05.06.87**

(54) **Produit cosmétique destiné à remettre la peau en bon état.**

(43) Date de publication de la demande:
**07.12.88 Bulletin 88/49**

(45) Mention de la délivrance du brevet:
**10.04.91 Bulletin 91/15**

(84) Etats contractants désignés:
**AT BE CH DE ES FR GB GR IT LI LU NL SE**

(56) Documents cités:
DE-A- 1 467 783    FR-A- 1 209 935
FR-A- 1 599 060    FR-A- 2 461 744
FR-A- 2 573 651    FR-A- 2 591 104
FR-A- 2 591 112    FR-M- 2 330

(73) Titulaire: **LABORATOIRES URGO**
**42 Rue de Longvic**
**F-21300 Chenove(FR)**

(72) Inventeur: **Desjonqueres, Stéphane**
**7 rue du Chant des Oiseaux**
**Montesson Les Yvelines(FR)**

(74) Mandataire: **Cabinet Pierre HERRBURGER**
**115, Boulevard Haussmann**
**F-75008 Paris(FR)**

## Description

La présente invention concerne un produit cosmétique destiné à réparer les peaux fragilisées des personnes se trouvant en position allongée pendant des périodes prolongées.

Les affections cutanées des malades alités, dont le stade final est l'escarre, sont une véritable hantise pour le personnel hospitalier.

L'escarre est, en effet, une nécrose de la peau (derme, épiderme, muscle) qui est créée par un manque de vascularisation, c'est-à-dire un infarctus de la circulation sanguine superficielle, aussi bien des capillaires que des veines et des artères. Ce phénomène est dû à la compression et au "tassement" de la surface cutanée du patient sur la surface sur laquelle il est couché, par suite de son propre poids.

Lorsque l'escarre se trouve au stade terminal, on note, par suite, de l'absence de vascularisation susmentionnée, un pourrissement en surface de la peau qui entraîne la formation de "trous" qui se propagent sur l'épiderme, le derme, la couche graisseuse, les muscles et les os du patient.

Il est bien connu que les problèmes d'escarres sont de nature à augmenter notablement (jusqu'à doubler) les temps d'hospitalisation nécessaires pour une affection déterminée.

Il est, par suite, nécessaire de rechercher des moyens efficaces de nature à empêcher, ou tout au moins, à freiner ces affections cutanées.

Dans ce but, on utilise classiquement dans les hôpitaux des moyens mécaniques tels que secouage, matelas liquides... Ces moyens s'avèrent toutefois chers et peu efficaces : ils ne sont, en effet, pas de nature à empêcher une "fragilisation" de la peau des malades devant rester en position immobile, qui correspond, en fait, au stade initial de l'escarre et constitue donc une menace potentielle ; celle-ci se concrétise par une modification superficielle de la peau qui devient rose, se desquame douloureusement et tombe en lambeaux. Ce phénomène est généralement accompagné ou suivi de "phlyctene" c'est-à-dire de formation de cloques.

Le traitement traditionnel de ces problèmes consiste en des massages vigoureux de pétrissement dits "trophiques" dont le but est de tenter de rétablir la circulation dans les paties nécrosées. Ces massages doivent être effectués pendant des périodes de 7 à 10 minutes, au moins, deux fois par jour, ce qui est particulièrement contraignant pour le personnel hospitalier et particulièrement désagréable pour les malades.

Pour faciliter ces massages, on a déjà proposé des produits tels que l'éosine, qui peuvent avoir l'avantage de faciliter le glissement de la main du practicien sur la peau du malade, mais se sont malheureusement révélés sans aucun effet sur l'affection cutanée proprement dite.

La présente invention a pour objet de proposer un produit cosmétique destiné à réparer les peaux fragilisées des personnes se trouvant allongées pendant des périodes prolongées qui est syscceptible d'améliorer notablement les résultats obtenus avec les massages susmentionnés, et, dans certains cas, de se substituer totalement de ceux-ci.

A cet effet, l'invention concerne un produit cosmétique caractérisé en ce qu'il contient de l'huile de maïs hyperoxygénée par saturation en oxygène et exposition intensive et contrôlée aux ultraviolets de façon à présenter un taux de peroxydation compris entre 50 et 120 exprimé en milli-équivalents et une teneur en glycérides oxydés comprise entre 5 et 40, la définition de cette huile en spectrophotométrie UV étant de 1 à 6 pour le facteur E 270 et de 8 à 60 pour le facteur E 232.

Cette définition correspond à la norme française NF T 60 223 qui s'applique à la détermination de l'absorbance spécifique en rayonnement ultraviolet des corps gras d'origine animale et végétale.

On s'est aperçu de manière surprenante que les huiles définies ci-dessus avaient une action particulièrement avantageuse, sur les peaux fragilisées des personnes allongées ; il s'est, en effet, avéré qu'une application légère cosmétique est de nature à se substituer en quelques secondes au massage vigoureux classique avec des résultats cliniques équivalents et même supérieurs.

On a pu, également, constater qu'un traitement entre 6 et 30 jours permettait de restituer le peau à un état lisse et ferme, ce qui constitue un avantage considérable pour le personnel hospitalier, d'autant plus si l'on tient compte du fait que 15 % des malades hospitalisés sont menacés d'escarres.

Selon une autre caractéristique de l'invention, le produit comporte entre 0,5 et 3 %, de préférence environ 1 %, d'une essence aromatique, notamment d'une essence aromatique à base d'anis.

Le rôle de cette essence est de masquer l'odeur de l'huile de maïs qui est très désagréable et peut être incommodante aussi bien pour les malades que pour les infirmiers chargés d'effectuer les massages. Le choix d'une essence alimentaire permet de satisfaire dans tous les cas aux réglementations du point de vue de la toxicité.

Le produit cosmétique objet de l'invention peut, selon les cas, se présenter soit sous la forme d'une huile, soit sous la forme d'un gel. Dans le cas d'une huile, celle-ci contient, de préférence, environ 99 % d'huile de maïs hyperoxygénée et environ 1 % d'une essence aromatique notamment d'une essence aromatique à base d'anis.

Dans le cas d'un gel, celui-ci contient, de préférence, environ 93 % d'huile de maïs hyperoxygénée, 6 % d'un excipient notamment d'un excipient à base de silice et 1 % d'une essence aromatique,

notamment d'une essence aromatique à base d'anis.

En tant qu'excipient, on peut, par exemple, mentionner le produit commercialisé par les Laboratoires DEGUSSA FRANCE à Neuilly sur Seine, sous la dénomination AEROSOL 300.

L'activité du produit cosmétique conforme à l'invention sera mise en lumière grâce aux exemples ci-dessous :

EXEMPLE 1 :

Quatre malades alités, dont un présentait au commencement de l'expérimentation, un début d'escarre sacré et dont deux présentaient, au niveau des mollets et des pieds, une affection de la peau à type de desquamation sur des débuts d'atteintes artéritiques, ont été traités par le produit cosmétique conforme à l'invention.

Les séances de traitement avec le produit ont été effectuées, en moyenne, toutes les quatre heures ; ces séances ont, tout d'abord été mal supportées par les patients, même si ceux-ci comprenaient bien le but de ces massages ; ils étaient, d'ailleurs, d'autant plus mal supportés que la peau était plus fragilisée. A l'issue de quelques séances de massage avec le produit cosmétique conforme à l'invention, sous forme d'huile, les malades ont néanmoins reconnu que le soin devenait plus agréable.

A la fin du traitement, l'état dermatologique des quatre patients était nettement amélioré.

L'essai de la composition sur les deux malades présentant une affection de la peau a donné un résultat très rapide après cinq ou six applications : chute des "écailles", restitution d'une peau lisse et nette et, cependant, ferme et solide.

En conclusion, les expérimentation susmentionnées ont abouti, dans tous les cas, à des résultats satisfaisants ; le produit respecte l'épiderme du patient et sa literie n'est pas souillée à l'issue de la séance de soin contrairement aux produits traditionnels liquides avec lesquels il est pratiquement impossible de ne pas mouiller les draps du malade.

EXEMPLE 2 :

Sur une étude portant sur 27 cas à la clinique de rééducation fonctionnelle IPOCA de CANNES, le Docteur RIGAL à démontré l'efficacité du produit dans les conditions de pré-escarre.

La répartition des âges était de :
- 10 malades de plus de 90 ans
- 11 malades de 80 à 90 ans,
- 5 malades de 70 à 80 ans,
- 1 malade de 66 ans.

La menace d'escarre se manifestait par un érythème prononcé dans les 27 cas, accompagné, dans 6 cas, par un halo ischémique et 6 cas, par un oedème dermo-épidermique.

L'application du produit était faite deux fois par jour par application légère d'une durée moyenne de 30 secondes.

Les localisation étaient les suivantes :

- région occipitale    2
- coude                2
- sacrée              12
- ischiatique          1
- fessière            23
- talonnière          11.

Le taux de guérison a été de 100 %, restitution d'une peau nette, lisse et ferme.
pour deux malades - après 4 jours,
pour six malades - après 6 jours,
pour quatre malades - après 8 jours,
pour sept malades - après 10 jours,
pour huit malades - après 15 jours.

**Revendications**

1. Produit cosmétique destiné à réparer les peaux fragilisées des personnes se trouvant en position allongée pendant des périodes prolongées, caractérisé en ce qu'il contient de l'huile de maïs hyperoxygénée par saturation en oxygène et exposition intensive et contrôlée aux ultraviolets de façon à présenter un taux de péroxydation compris entre 50 et 120 exprimé en milli-équivalents et une teneur en glycérides oxydés comprise entre 5 et 40, la définition de cette huile en spectrophotométrie UV étant de 1 à 6 pour le facteur E 270 et de 8 à 60 pour le facteur E 232.

2. Produit cosmétique selon la revendication 1, caractérisé en ce qu'il comporte entre 0,5 et 3 %, de préférence environ 1 % d'une essence aromatique, notamment d'une essence aromatique à base d'anis.

3. Produit cosmétique selon l'une quelconque des revendications 1 et 2, caractérisé en ce qu'il se présente sous la forme d'une huile.

4. Produit cosmétique selon la revendication 3, caractérisé en ce qu'il contient environ 99 % d'huile de maïs hyperoxygénée et environ 1 % d'une essence aromatique, notamment d'une essence aromatique à base d'anis.

5. Produit cosmétique selon l'une quelconque des revendications 1 et 2, caractérisé en ce qu'il se présente sous la forme d'un gel.

6. Produit cosmétique selon la revendication 5, caractérisé en ce qu'il contient environ 93 % d'huile de maïs hyperoxygénée, 6 % d'un excipient, notamment d'un excipient à base de silice et 1 % d'une essence aromatique, notamment d'une essence aromatique à base d'anis.

## Claims

1. Cosmetic product intended for repairing skin which has become susceptible to damage in individuals maintaining a recumbent position for prolonged periods, characterised in that it contains maize oil hyperoxygenated by saturation with oxygen and intensive and controlled exposure to ultraviolet rays so as to possess a degree of peroxidation of between 50 and 120 expressed in milliequivalents, and a content of oxidised glycerides of between 5 and 40, the definition of this oil in UV spectrophotometry being from 1 to 6 for the factor E 270 and from 8 to 60 for the factor E 232.

2. Cosmetic product according to Claim 1, characterised in that it contains between 0.5 and 3%, and preferably approximately 1%, of an aromatic essential oil, in particular an aromatic essential oil based on aniseed.

3. Cosmetic composition according to either of Claims 1 and 2, characterised in that it takes the form of an oil.

4. Cosmetic product according to Claim 3, characterised in that it contains approximately 99% of hyperoxygenated maize oil and approximately 1% of an aromatic essential oil, in particular an aromatic essential oil based on aniseed.

5. Cosmetic product according to either of Claims 1 and 2, characterised in that takes the form of a gel.

6. Cosmetic product according to Claim 5, characterised in that it contains approximately 93% of hyperoxygenated maize oil, 6% of an excipient, in particular an excipient based an silica, and 1% of an aromatic essential oil, in particular an aromatic essential oil based on aniseed.

## Ansprüche

1. Kosmetisches Produkt zur Wiederherstellung der empfindlichen Haut von Personen, die sich während langer Zeit in liegender Stellung befinden, dadurch gekennzeichnet, daß es durch Sättigung mit Sauerstoff und intensive und kontrollierte Einwirkung ultravioletter Strahlen derart hyperoxydiertes Maisöl enthält, daß es einen Peroxydationsanteil zwischen 50 und 120 in Milliäquivalent aufweist und einen Gehalt an oxydierten Glyzeriden zwischen 5 und 40, wobei die UV-Spektralphotometrische Definition dieses Öls zwischen 1 und 6 für den Faktor E 270 und zwischen 8 und 60 für den Faktor E 232 ist.

2. Kosmetisches Produkt gemäß Patentanspruch 1, dadurch gekennzeichnet, daß es zwischen 0,5 und 3%, vorzugsweise 1% einer aromatischen Essenz enthält, insbesondere einer aromatischen Essenz auf Anisbasis.

3. Kosmetisches Produkt gemäß irgendeinem der Patentansprüche 1 und 2, dadurch gekennzeichnet, daß es die Form eines Öles hat.

4. Kosmetisches Produkt gemäß Patentanspruch 3, dadurch gekennzeichnet, daß es ungefähr 99% hyperoxydiertes Maisöl und 1% einer aromatischen Essenz, insbesondere einer aromatischen Essenz auf Anisbasis, enthält.

5. Kosmetisches Produkt gemäß irgendeinem der Patentansprüche 1 und 2, dadurch gekennzeichnet, daß es die Form eines Gels hat.

6. Kosmetisches Produkt gemäß Patentanspruch 5, dadurch gekennzeichnet, daß es ungefähr 93% hyperoxydiertes Maisöl, 6% eines Arzneiträgers insbesondere eines Arzneiträgers auf Siliziumdioxidbasis, und 1% einer aromatischen Essenz, insbesondere einer aromatischen Essenz auf Anisbasis, enthält.